# EUROPEAN PATENT SPECIFICATION

(11) **EP 2 155 296 B1**
(45) Date of publication and mention of the grant of the patent: **28.08.2019**
(21) Application number: 08761210.7
(22) Date of filing: 19.06.2008
(51) Int. Cl.: A61M 5/158

(54) **CANNULA INSERTION DEVICE WITH AUTOMATIC NEEDLE RETRACTION COMPRISING ONLY ONE SPRING**
KANÜLENEINFÜHRGERÄT MIT AUTOMATISCHEM NADELRÜCKZUG WELCHES NUR EINE FEDER BEINHALTET
DISPOSITIF D'INSERTION POUR AIGUILLE AVEC RETRACTION AUTOMATIQUE DE L'AGUILLE COMPRENANT SEULEMENT UN RESSORT

(30) Priority: 20.06.2007 US 945172 P
(43) Date of publication of application: 24.02.2010
(73) Proprietor: Unomedical A/S, 3460 Birkerød (DK)
(72) Inventor: HASTED, Søren, Bo, DK-2100 København Ø (DK)
(74) Representative: Campabadal i Monfà, Gemma
(86) International application number: PCT/EP2008/057769
(87) International publication number: WO 2008/155377

(56) References cited:
- WO-A-2006/061354
- US-A1- 2004 158 207
- US-A1- 2007 093 754

## Description

The invention relates to an inserter for a medical device e.g. an infusion set for intermittent or continuous administration of a therapeutical substance, such as e.g. insulin. The inserter comprises an insertion needle and a spring unit assuring automatic insertion and automatic retraction of the insertion needle.

### Background of the invention

It is known to construct inserters for infusion sets which hides and protects the insertion needle before insertion and which retracts the insertion needle after penetration of the patients skin and thereafter hides and protects the insertion needle.

Such a device is known from EP 1.762.259. The inserter according to this document comprises a needle hub comprising an insertion needle and two spring units assuring automatic insertion and automatic retraction of the insertion needle. Although the design of the device is compact and user friendly the mechanism is relatively complex as two spring units are used in order to make the device work correctly.

From US 2004/0158207 (Hunn et al.) is known a device for inserting a cannula into tissue, including a cannula (3), a protective element surrounding and accommodating the cannula and insertion needle before and after insertion, an operating element for moving the cannula out of the protective element, and a holder fixedly connected to the cannula. The spring (31) in the automatic embodiments (fig. 13-18) of this document, pushes directly on the needle carrier (34) when the needle carrier is brought to a retracted position.

From WO 2006/061354 (Novo Nordisk A/S) is known a medical device comprising a transcutaneous device unit and a process unit. The unit is adapted to be mounted to a skin surface of a subject and comprises a first housing, a transcutaneous device, and may comprise a flexible patch portion with an upper surface and a lower mounting surface adapted for application to the skin of a subject. The process unit comprises a second housing with a lower surface and a process assembly. The first and second housings are adapted to be secured to each other in such a way that the lower surface of the second housing is allowed to move freely relative to at least a portion of the underlying skin surface or patch. In this way a relatively flexible patch porion can adapt to the skin surface to which it is mounted both statically and dynamically without being restricted in its movements by the normally much stiffer process unit. Figs. 25A-25D shows a schematic illustration of a multistep concept with a single loaded spring (1910) used to insert a cannula. When a first locking structure is released the cannula together with the insertion needles is inserted and a second locking structure (1912) is ready to be disengaged. When the second locking structure is disengaged the insertion needle is pulled away from the subject, and a third locking structure (1913) is ready to be disengaged. When the third locking structure is disengaged the cannula is pulled out. I.e. it is the intention that the inserter stays at the insertion position until the cannula has to be removed.

The present invention provides both protection of the insertion needle before insertion and after retraction and at the same time the inserter device is of a simple construction which only needs one spring unit.

### Description of invention

The object of the invention is to provide a simple, non-expensive inserter for an infusion device which inserter would be easy and safe for the user to handle during use and safe to dispose of after use.

The invention concerns an inserter for inserting a medical device comprising a housing, a first body which is movable relative to the housing and comprising penetrating means pointing in the direction of insertion, a second body which is also movable relative to the housing and driving means which move respectively the first body and the second body relative to the housing wherein the driving means moves the first body in the direction of insertion and moves the second body in a direction different from the insertion direction.

In one embodiment the driving means first move the first body in the direction of insertion and then moves the second body in the direction different from the insertion direction. The driving means can comprise a single spring unit which could be a cluster of several springs or a single spring unit, e.g. a coiled spring. The spring unit of this one embodiment can work by first expanding in the insertion direction and then expanding in the direction different from the insertion direction. This direction different from the insertion direction can be in an angle of 180° ± 5° to the insertion direction.

In one embodiment the inserter device can be provided with first locking means which locking means can keep the first body in a chosen retracted position in relation to the housing while the driving means are biased i.e. the driving means posses stored or potential energy. These first locking means can comprise a protruding part on the first body which interacts with an opening in the housing.

In one embodiment the inserter is provided with second locking means which second locking means can keep the second body in a chosen forward position in relation to the housing while the driving means are biased i.e. the driving means posses potential energy, e.g. the second locking means comprises an inwardly protruding part of the housing which interacts with a distally turned surface of the second body.

According to one embodiment the inserter comprises means for locking the penetrating means to the second body while the second body is moving from a forward to a retracted position in relation to the housing.

The medical device used with the inserter device can be e.g. an infusion part for administrating medication or a sensor device for measuring values e.g. of the blood or a simple port/gateway for administering medication by a syringe or the like.

The invention also relates to a process for positioning a medical device on the skin of a patient which process comprises the following steps:
a) removing any packing and preparing the skin adhesion of the medical device;
b) placing the proximal end of the inserter against the skin of the patient or against a pre-positioned pad;.
c) unreleasing a first set of locking means (9) which will bring a first body (1) to reach a forward position and cause a proximal surface of the medical device to reach the surface it is to be placed on;
d) unreleasing a second set of locking means (11) which will result in that a second body (4) including parts locked to the second body (4) are distanced from the medical device which is left on the patients skin;
e) removing the inserter from the position while the medical device is left in the position.

### Description of the drawings

The invention is explained in greater detail below with reference to the accompanying drawings wherein preferred embodiments of the invention is shown.
Fig. 1 shows a cut-through view of an embodiment of a device according to the invention. The embodiment is in a first state where the penetrating member is retracted and the spring unit is tightened.
Fig. 2 shows the same embodiment as fig. 1 in the same state but in a view which is perpendicular to the view of fig. 1.
Fig. 3 shows the same embodiment as fig. 1 and 2 in a second state where the penetrating member is in a forward position and the spring unit in a semi-tightened state.
Fig. 4 shows the same embodiment as the previous figures in a third state where the penetrating member is in a retracted position; the medical device is left on the skin of the patient and the spring unit in a non-tightened state.
Fig. 5 shows an exploded view of the same embodiment as the previous figures.

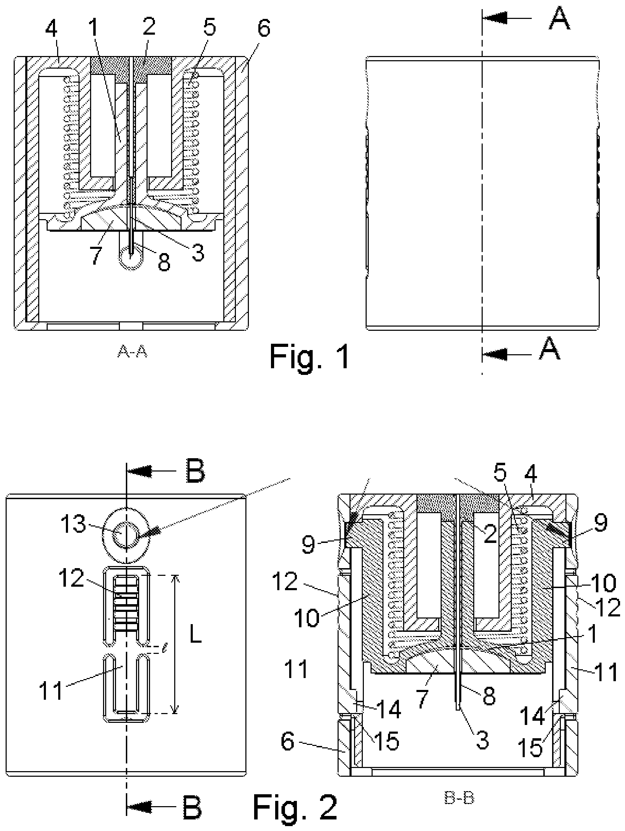

The device illustrated in fig. 1 comprises a carrier body 1 for a medical device to be inserted, a needle hub 2 comprising a penetrating member 3, a movable part 4, a spring unit 5 and a housing 6. The spring unit 5 is tightened or biased in the space between the first body 1 and the second body 4, both of which are located in the housing 6.,

The medical device can be al sorts of devices which for some reason needs to be placed sub- or transcutaneously on a patient for a shorter or longer time. In the embodiment illustrated in fig. 1-5 the medical device is a port-device. A port-device is a device which is placed on a user's skin for a period of upto three days and replaces numerous injections normally made be a syringe. The medical device could instead be an infusion part which device is also normally situated on the patient for several days and an infusion part has means for connecting it to a delivery device e.g. for delivering insulin. An infusion part can e.g. provide an optimized administration of a therapeutic substance as it allows very small doses to be transferred often. The medical device can also be a sensor device provided with a subcutaneously placed sensor which is in contact with the patient's blood and able to register desired elements in the blood e.g. the sugar level.

The port-device shown in the figures comprises a body 7 having a through going opening and a cannula 8 made of a soft and flexible material.

Fig. 2 shows the same embodiment in the same state as fig. 1 but from an angle perpendicular to the view angle illustrated in fig. 1. From this angle it is possible to see how the carrier body 1 is provided with two protruding locking parts 9 which are positioned at the end of each a flexible arm 10. The protruding locking parts 9 fit into corresponding openings in the stationary housing 6 and lock the carrier body 1 in a retracted position before use. The spring unit 5 is biased and pushes against the locked carrier body 1 (downwards on fig. 2) and the movable part 4 (upwards on fig. 2). When a user puts pressure on the opposite positioned pressure points 13 which are corresponding to the oppositely positioned protruding parts 9, the protruding parts 9 are disengaged from the openings in the housing 6 and the spring unit 5 will push the carrier body 1 downwards as the movable part 4 is still locked in relation to the housing 6 with secondary locking means.

The secondary locking means have the form of a longitudinal cut-out 11 having the length L. The longitudinal cut-out is fixed to the housing 6 by thin parts of material close to the middle section, the fastening parts have the length/width 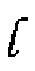 and are symmetrically placed on each side of the cut-out 11. The length/width of the fastening parts are less than 10% of the total length of the cut-out 11, in the present embodiment the length/width of the fastening parts are approximately 6% of the length of the cut-out 11. The fastening parts are so relatively thin that it is possible to pivot the free ends of the cut-out 11 around the axis formed by the fastening parts. A first of the free ends 12 is provided with means which will make the user recognize these parts of the cut-out 11 as pressure points. In the embodiment in fig. 2 these means comprises a series of flutes which can be felt by the user and recognized as a pressure point. When the user put pressure on the two opposite positioned first free ends 12, the first free ends 12 move towards the centre of the device while the second free ends moves outwards away from the centre of the device. The second free ends are provided with inwardly protruding parts 14 which are engaged with the edge 15 of an opening in the movable part 4.

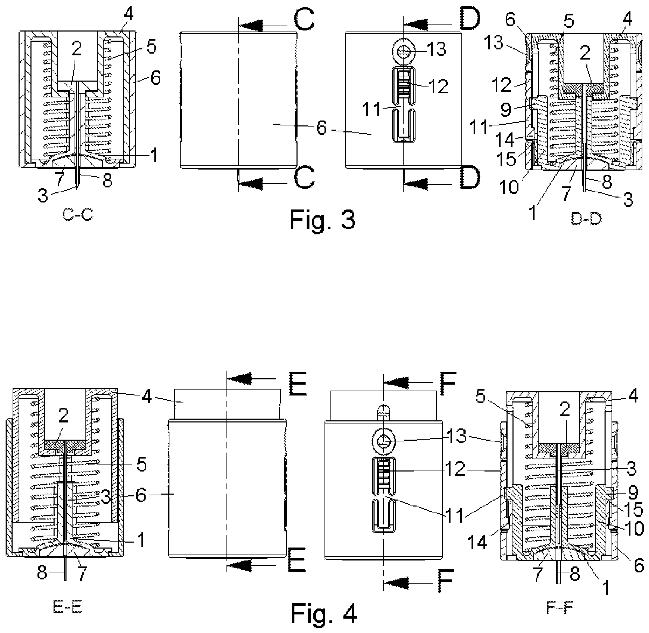

Fig. 3 shows two perpendicular views of the inserter device, the view C-C are similar to fig. 1 and the view D-D is similar to fig. 2. The spring unit 5 of the embodiment in fig. 3 is in a semi-biased state, i.e. the spring unit 5 is released from the first lock made between the protruding parts 9 and the housing 6 and as a result of the releasing of the first locking means the cannula 8 of the medical device has been inserted subcutaneously in the patient. Also the carrier body 1 and the needle hub 2 has moved in relation to the movable part 4, the carrier body 1 has been distanced from the movable part 4 while the needle hub 2, which moves together with the carrier body 1, is moved from a surface position to an inner position in relation to the movable part 4 which makes it possible for the user to register that the penetrating member 3 is now in an inserted position.

Fig. 4 shows the same perpendicular views of the same embodiment of the inserter device as shown in fig. 3, but the spring unit 5 of the embodiment in fig. 4 is in an un-biased state, i.e. the spring unit 5 is also released from the second lock made between the inwardly protruding parts 14 of the housing 6 and the movable part 4 and as a result of the releasing of the second locking means the needle hub 2 together with the carrier body 1 is retracted from the patient. The user can observe this as the movable part 4 is raised in relation to the stationary housing 6.

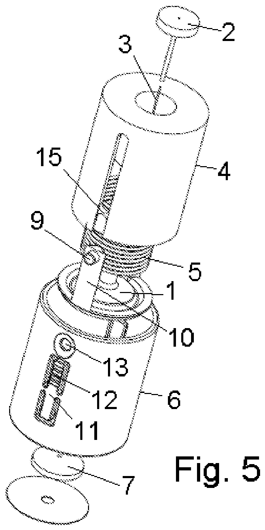

Fig. 5 shows the same embodiment as fig. 1-4 but in an exploded view where the parts are partly separated in order to show the individual parts in a three-dimensional way and how the individual parts relate to each other. The parts have the same reference numbers as in fig. 1-4.

The inserter device is normally delivered to the user joined with the medical device in a sterile packing which has to be removed before use. Either the medical device is provided with a mounting pad, that is a mounting pad is unreleasably fastened to the proximal side of the medical device, or a mounting pad is provided in a separate sterile packing and placed on the skin of the patient at a suitable insertion place before the medical device is inserted. If the mounting pad is unreleasably fastened to the medical device it will normally be necessary to remove a release layer from the adhesive surface of the mounting pad before using the inserter device.

After the sterile packing is removed and either the release layer is removed or the separate mounting pad is positioned, the inserter device is ready for insertion of the medical device.
- The proximal end of the inserter device is then placed against the skin of the patient or against the pre-positioned mounting pad; the proximal end of the inserter device is the end towards which the sharp end of the penetrating member points.
- Then the user puts pressure on the oppositely positioned pressure points 13 which will unlock the first locking means, i.e. the protruding locking parts 9. The unlocking of the first locking means result in the carrier body 1 including the medical device and the needle hub 2 being moved forward at the speed provided by the spring unit 5. The movement is stopped as the medical device touches the skin of the patient as further extension of the spring unit 5 is then prevented.
- Then the user puts pressure on the oppositely positioned pressure points 12 which unlocks the second locking means, i.e. the pivotally fastened cut-out 11. The unlocking of the second locking means result in the movable part 4 including the needle hub 2 being pushed away from the carrier body 1 and the medical device which is left inserted into the patients skin.
- The inserter device can now be removed from the insertion position while the medical device is left in the inserted position.

## Claims

1. An inserter device for sub- or transcutaneous placement of a medical device comprising
- a housing (6),
- a carrier body (1) for the medical device to be inserted which is movable relative to the housing and comprising a needle hub (2) carrying penetrating means (3) pointing in the direction of insertion,
- a second body (4) which is also movable relative to the housing (6) and
- driving means (5) which move respectively the carrier body (1) and the second body (4) relative to the housing (6)
wherein the driving means comprise a single spring unit (5) placed concentrically between the carrier body (1) and the second body (4), which driving means first moves the carrier body (1) in the direction of insertion and then moves the second body (4) in a direction which is in an angle of 180° ± 5° to the insertion direction, thereby retracting the second body (4)
from the carrier body (1) after insertion of the medical device, and wherein the inserter device comprises means for locking the penetrating means to the second body while the second body is moving from a forward to a retracted position in relation to the housing, wherein the carrier body (1) is provided with first locking means (9) keeping the carrier body (1) in a chosen retracted position in relation to the housing (6) while the driving means (5) are biased and in that the inserter device is provided with second locking means (11, 14) which second locking means keeps the second body (4) in said chosen forward position in relation to the housing (6) while the driving means (5) are biased before insertion of the medical device.

2. An inserter according to claim 1, **characterized in that** the spring unit (5) is left in an un-biased state after having moved the second body (4).

3. An inserter according to claim 1 or 2, **characterized in that** the spring unit (5) is a coiled spring.

4. An inserter according to any of claims 1-3, wherein the carrier body (1) is provided with two oppositely positioned protruding locking parts (9) which are positioned at the end of each a flexible arm (10), the protruding locking parts (9) fit into corresponding openings in the housing (6) and thereby lock the carrier body (1) in a retracted position before use.

5. An inserter according to claim 4, wherein the housing (6) is provided with two oppositely positioned pressure points (13) corresponding to the oppositely positioned protruding parts (9), and when a user puts pressure on the opposite positioned pressure points (13), the protruding parts (9) disengage from the openings in the housing (6) and the spring unit (5) push the carrier body (1) downwards.

6. An inserter according to claim 5, **characterized in that** the second locking means comprises an inwardly protruding part (14) of the housing (6) which interacts with a distally turned surface (15) of the second body (4).

7. An inserter according to claim 5, wherein the second locking means have the form of a longitudinal cut-out (11) having the length L which longitudinal cut-out is fixed to the housing (6) by thin parts of material close to the middle section in such a way that it is possible to pivot the free ends of the cut-out (11) around the axis formed by the fastening parts, the fastening parts have the length/width 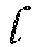 (and are symmetrically placed on each side of the cut-out (11).

8. An inserter according to claim 7, wherein the length/width of the fastening parts are less than 10% of the total length of the cut-out (11).

9. An inserter according to claim 8, wherein a first (12) of the free ends is provided with means which will make the user recognize these parts of the cut-out(11)as pressure points which can be felt by the user and recognized as a pressure point.

10. An inserter according to any of the claims 1-9, **characterized in that** the inserter comprises means for locking the penetrating means (3) to the second body (4) while the second body (4) is moving from a forward to a retracted position in relation to the housing (6).

11. An inserter according to any of the preceding claims, **characterized in that** the medical device is an infusion part for administrating medication or a sensor device for measuring values or a simple port/gateway for administering medication by a syringe or the like.

12. An inserter according to any of the preceding claims, wherein pressure points (13) corresponding to the oppositely positioned protruding parts (9) and used to disengage the carrier body (1) from the housing (6), are different from pressure points (12) used to disengage the second body (4) from the housing (6).

13. An inserter according to any of the preceding claims, wherein unlocking of the second locking means result in the second body (4) including the needle hub (2) being pushed away from the carrier body (1) and the medical device.

14. Process for positioning a medical device on a surface using an inserter **characterized in that** the process comprises the following steps:
a) providing the inserter device according to any of the proceeding claims;
b) placing a proximal end of the inserter against the surface or against a pre-positioned pad;.
c) unreleasing a first set of locking means (9) which brings a first body (1) to reach a forward position and cause the proximal end of the medical device to reach the surface it is to be placed on;
d) unreleasing a second set of locking means (11) which results **in that** a second body (4) including parts locked to the second body (4) are distanced from the medical device which is left on the surface;
e) removing the inserter from the medical device which is left on the surface.

## Patentansprüche

1. Inserter-Vorrichtung zur sub- oder transkutanen Anordnung einer medizinischen Vorrichtung, umfassend
- ein Gehäuse (6),
- einen Trägerkörper (1) für die einzuführende medizinische Vorrichtung, der relativ zum Gehäuse beweglich ist und einen Nadelansatz (2) umfasst, der in Einführrichtung weisende Durchdringungsmittel (3) trägt,
- einen zweiten Körper (4), der ebenfalls relativ zum Gehäuse (6) beweglich ist und
- Antriebsmittel (5), die jeweils den Trägerkörper (1) und den zweiten Körper (4) relativ zum Gehäuse (6) bewegen
wobei die Antriebsmittel eine einzige Federeinheit (5) umfassen, die konzentrisch zwischen dem Trägerkörper (1) und dem zweiten Körper (4) angeordnet ist, wobei die Antriebsmittel den Trägerkörper (1) zuerst in Einführrichtung und dann den zweiten Körper (4) in einer Richtung bewegen, die in einem Winkel von 180° ± 5° zur Einführrichtung liegt, wodurch der zweite Körper (4) nach dem Einführen der medizinischen Vorrichtung aus dem Trägerkörper (1) zurückgezogen wird, und wobei die Inserter-Vorrichtung Mittel zum Verriegeln der Durchdringungsmittel in den zweiten Körper umfasst, während sich der zweite Körper in Bezug auf das Gehäuse von einer vorderen in eine zurückgezogene Position bewegt,
wobei der Trägerkörper (1) mit ersten Verriegelungsmitteln (9) versehen ist, die den Trägerkörper (1) in einer ausgewählten zurückgezogenen Position in Bezug auf das Gehäuse (6) halten, während die Antriebsmittel (5) vorgespannt sind, und dadurch, dass die Inserter-Vorrichtung mit zweiten Verriegelungsmitteln (11, 14) versehen ist, die den zweiten Körper (4) in der gewählten vorderen Position in Bezug auf das Gehäuse (6) halten, während die Antriebsmittel (5) vor dem Einführen der medizinischen Vorrichtung vorgespannt sind.

2. Inserter nach Anspruch 1, **dadurch gekennzeichnet, dass** die Federeinheit (5) nach dem Bewegen des zweiten Körpers (4) in einem nicht vorgespannten Zustand belassen wird.

3. Inserter nach Anspruch 1 oder 2, **dadurch gekennzeichnet, dass** die Federeinheit (5) eine Schraubenfeder ist.

4. Inserter nach einem der Ansprüche 1-3, wobei der Trägerkörper (1) mit zwei gegenüberliegend positionierten vorstehenden Verriegelungsteilen (9) versehen ist, an deren Enden jeweils ein flexibler Arm (10) positioniert ist, wobei die vorstehenden Verriegelungsteile (9) in entsprechende Öffnungen im Gehäuse (6) passen und dadurch den Trägerkörper (1) vor Gebrauch in einer zurückgezogenen Position verriegeln.

5. Inserter nach Anspruch 4, wobei das Gehäuse (6) mit zwei gegenüberliegenden Druckpunkten (13) versehen ist, die den gegenüberliegenden vorstehenden Teilen (9) entsprechen, und wenn ein Benutzer auf die gegenüberliegenden Druckpunkte (13) Druck ausübt, sich die vorstehenden Teile (9) aus den Öffnungen im Gehäuse (6) lösen und die Federeinheit (5) den Trägerkörper (1) nach unten drücken.

6. Inserter nach Anspruch 5, **dadurch gekennzeichnet, dass** das zweite Verriegelungsmittel einen nach innen vorstehenden Teil (14) des Gehäuses (6) umfasst, der mit einer distal gedrehten Oberfläche (15) des zweiten Körpers (4) zusammenwirkt.

7. Inserter nach Anspruch 5, wobei die zweiten Verriegelungsmittel die Form eines Längsausschnitts (11) mit der Länge L haben, wobei der Längsausschnitt durch dünne Materialteile nahe dem Mittelabschnitt derart am Gehäuse (6) befestigt ist, dass es möglich ist, die freien Enden des Ausschnitts (11) um die durch die Befestigungsteile gebildete Achse zu schwenken, wobei die Befestigungsteile die Länge/Breite haben und symmetrisch auf jeder Seite des Ausschnitts (11) angeordnet sind.

8. Inserter nach Anspruch 7, wobei die Länge/Breite der Befestigungsteile weniger als 10 % der Gesamtlänge des Ausschnitts (11) beträgt.

9. Inserter nach Anspruch 8, wobei ein erstes (12) der freien Enden mit Mitteln versehen ist, die den Benutzer diese Teile des Ausschnitts (11) als Druckpunkte erkennen lassen, die vom Benutzer gefühlt und als ein Druckpunkt erkannt werden können.

10. Inserter nach einem der Ansprüche 1-9, **dadurch gekennzeichnet, dass** der Inserter Mittel zum Verriegeln der Durchdringungsmittel (3) an dem zweiten Körper (4) umfasst, während sich der zweite Körper (4) in Bezug auf das Gehäuse (6) von einer vorderen in eine zurückgezogene Position bewegt.

11. Inserter nach einem der vorstehenden Ansprüche, **dadurch gekennzeichnet, dass** die medizinische Vorrichtung ein Infusionsteil zur Verabreichung von Medikamenten oder eine Sensorvorrichtung zur Messung von Werten oder ein einfacher Port/Zugang zur Verabreichung von Medikamenten durch eine Spritze oder dergleichen ist.

12. Inserter nach einem der vorstehenden Ansprüche, wobei die Druckpunkte (13), die den gegenüberliegenden vorstehenden Teilen (9) entsprechen und zum Lösen des Trägerkörpers (1) vom Gehäuse (6) verwendet werden, andere sind als die Druckpunkte (12), die zum Lösen des zweiten Körpers (4) vom Gehäuse (6) verwendet werden.

13. Inserter nach einem der vorstehenden Ansprüche, wobei das Entriegeln der zweiten Verriegelungsmittel dazu führt, dass der zweite Körper (4) einschließlich des Nadelansatzes (2) vom Trägerkörper (1) und der medizinischen Vorrichtung weggeschoben wird.

14. Verfahren zum Positionieren einer medizinischen Vorrichtung auf einer Oberfläche unter Verwendung eines Inserters, **dadurch gekennzeichnet, dass** das Verfahren die folgenden Schritte umfasst:
a) Bereitstellen der Inserter-Vorrichtung nach einem der vorstehenden Ansprüche;
b) Anordnen eines proximalen Endes des Inserters gegen die Oberfläche oder gegen ein vorpositioniertes Kissen;
c) Lösen eines ersten Satzes von Verriegelungsmitteln (9), die einen ersten Körper (1) dazu veranlassen, eine vordere Position zu erreichen, und das proximale Ende der medizinischen Vorrichtung dazu veranlassen, die Oberfläche zu erreichen, auf der sie angeordnet werden soll;
d) Lösen eines zweiten Satzes von Verriegelungsmitteln (11), was dazu führt, dass ein zweiter Körper (4), der Teile einschließt, die mit dem zweiten Körper (4) verriegelt sind, von der medizinischen Vorrichtung beabstandet ist, die auf der Oberfläche verbleibt;
e) Entfernen des Inserters von der medizinischen Vorrichtung, die auf der Oberfläche verbleibt.

## Revendications

1. Dispositif d'insertion pour placement sous- ou transcutané d'un dispositif médical, comprenant:
- un boîtier (6),
- un corps de support (1) pour le dispositif médical à insérer, qui est mobile par rapport au boîtier et comprend une garde d'aiguille (2) portant des moyens de pénétration (3) pointant dans la direction d'insertion,
- un second corps (4) qui est également mobile par rapport au boîtier (6) et
- des moyens d'entraînement (5) qui déplacent respectivement le corps de support (1) et le second corps (4) par rapport au boîtier (6),
dans lequel les moyens d'entraînement comprennent une unité élastique unique (5) placée concentriquement entre le corps de support (1) et le second corps (4), lesquels moyens d'entraînement déplacent d'abord le corps de support (1) dans la direction d'insertion et déplacent ensuite le second corps (4) dans une direction qui fait un angle de 180° ± 5° avec la direction d'insertion, rétractant de la sorte le second corps (4) du corps de support (1) après l'insertion du dispositif médical, et dans lequel le dispositif d'insertion comprend des moyens pour verrouiller les moyens de pénétration sur le second corps tandis que le second corps se déplace d'une position d'avancement à une position rétractée par rapport au boîtier,
dans lequel le corps de support (1) est pourvu de premiers moyens de verrouillage (9) maintenant le corps de support (1) dans une position rétractée choisie par rapport au boîtier (6) tandis que les moyens d'entraînement (5) sont sollicités et le dispositif d'insertion est pourvu d'un second moyen de verrouillage (11, 14), lequel second moyen de verrouillage maintient le second corps (4) dans ladite position d'avancement choisie par rapport au boîtier (6) tandis que les moyens d'entraînement (5) sont sollicités avant l'insertion du dispositif médical.

2. Dispositif d'insertion selon la revendication 1, **caractérisé en ce que** l'unité élastique (5) est laissée dans un état non sollicité après avoir déplacé le second corps (4).

3. Dispositif d'insertion selon la revendication 1 ou 2, **caractérisé en ce que** l'unité élastique (5) est un ressort hélicoïdal.

4. Dispositif d'insertion selon l'une quelconque des revendications 1 à 3, dans lequel le corps de support (1) est pourvu de deux parties de verrouillage saillantes (9) positionnées à l'opposé et qui sont disposées à l'extrémité de chaque bras souple (10), les parties de verrouillage saillantes (9) s'ajustent dans des ouvertures correspondantes du boîtier (6) et verrouillent de la sorte le corps de support (1) dans une position rétractée avant utilisation.

5. Dispositif d'insertion selon la revendication 4, dans lequel le boîtier (6) est pourvu de deux points de pression (13) positionnés à l'opposé et correspondant aux parties saillantes (9) positionnées à l'opposé et, lorsqu'un utilisateur applique une pression sur les points de pression positionnés à l'opposé (13), les parties saillantes (9) se dégagent des ouvertures du boîtier (6) et l'unité élastique (5) pousse le corps de support (1) vers le bas.

6. Dispositif d'insertion selon la revendication 5, **caractérisé en ce que** les seconds moyens de verrouillage comprennent une partie saillant vers l'intérieur (14) du boîtier (6) qui interagit avec une surface (15) du second corps (4) tournée vers la partie distale.

7. Dispositif d'insertion selon la revendication 5, dans lequel les seconds moyens de verrouillage se présentent sous la forme d'une découpe longitudinale (11) ayant la longueur L, laquelle découpe longitudinale est fixée au boîtier (6) par des parties de matériau minces proches de la section centrale de manière qu'il soit possible de faire pivoter les extrémités libres de la découpe (11) autour de l'axe formé par les parties de fixation, les parties de fixation ayant la longueur/largeur ℓ et étant placées de manière symétrique sur chaque côté de la découpe (11).

8. Dispositif d'insertion selon la revendication 7, dans lequel les longueur/largeur des parties de fixation sont inférieures à 10 % de la longueur totale de la découpe (11).

9. Dispositif d'insertion selon la revendication 8, dans lequel une première (12) des extrémités libres est pourvue de moyens qui feront que l'utilisateur reconnaîtra ces parties de la découpe (11) comme points de pression qui peuvent être ressentis par l'utilisateur et reconnus en tant que points de pression.

10. Dispositif d'insertion selon l'une quelconque des revendications 1 à 9, **caractérisé en ce que** le dispositif d'insertion comprend des moyens pour verrouiller les moyens de pénétration (3) sur le second corps (4) tandis que le second corps (4) se déplace d'une position d'avancement à une position rétractée par rapport au boîtier (6).

11. Dispositif d'insertion selon l'une quelconque des revendications précédentes, **caractérisé en ce que** le dispositif médical est une partie d'infusion pour l'administration d'un médicament ou un dispositif capteur pour mesurer des valeurs ou un simple orifice/une simple passerelle pour l'administration d'un médicament par une seringue ou analogue.

12. Dispositif d'insertion selon l'une quelconque des revendications précédentes, dans lequel les points de pression (13) correspondant aux parties saillantes positionnées à l'opposé (9) et utilisés pour dégager le corps de support (1) du boîtier (6), sont différents des points de pression (12) utilisés pour dégager le second corps (4) du boîtier (6).

13. Dispositif d'insertion selon l'une quelconque des revendications précédentes, dans lequel le déverrouillage des seconds moyens de verrouillage fait que le second corps (4) incluant la garde d'aiguille (2) est écarté du corps de support (1) et du dispositif médical.

14. Procédé de positionnement d'un dispositif médical sur une surface en utilisant un dispositif d'insertion, **caractérisé en ce que** le procédé comprend les étapes suivantes consistant à :
a) fournir le dispositif d'insertion selon l'une quelconque des revendications précédentes ;
b) placer une extrémité proximale du dispositif d'insertion contre la surface ou contre un pavé prépositionné ;
c) bloquer un premier ensemble de moyens de verrouillage (9), ce qui amène un premier corps (1) à atteindre une position d'avancement et amène l'extrémité proximale du dispositif médical à atteindre la surface sur laquelle il doit être placé ;
d) bloquer un second ensemble de moyens de verrouillage (11), ce qui fait qu'un second corps (4) comprenant des parties verrouillées sur le second corps (4) sont écartées du dispositif médical qui est laissé sur la surface ;
e) retirer le dispositif d'insertion du dispositif médical qui est laissé sur la surface.
